# EUROPEAN PATENT APPLICATION

(11) **EP 2 130 915 A1**
(43) Date of publication of application: **09.12.2009**
(21) Application number: 08720944.1
(22) Date of filing: 27.02.2008
(51) Int. Cl.: C12N 15/09, A61K 35/44, A61K 38/22, A61P 27/02, C12N 5/10, C12P 21/02

(54) **CELL CAPABLE OF EXPRESSING LACRITIN AT HIGH LEVEL**

(30) Priority: 28.02.2007 JP 2007049936
(71) Applicant: Senju Pharmaceutical Co., Ltd., Osaka-shi, Osaka 541-0046 (JP)
(72) Inventor: NAKAJIMA, Takeshi, Kobe-shi Hyogo 651-2241 (JP); AZUMA, Mitsuyoshi, Kobe-shi Hyogo 651-2241 (JP)
(74) Representative: Duckworth, Timothy John
(86) International application number: PCT/JP2008/053396
(87) International publication number: WO 2008/105454

(57) **Abstract**

The present invention relates to a means that allows supplying lacritin stably and in large amounts, and provides a vector containing polynucleotide that encodes lacritin, wherein said nucleotide is operatively linked to a cytomegalovirus immediate early enhancer/promoter and an SV40 late polyA signal, a lacritin-expressing cell prepared by introducing the vector into a cell, a method of producing recombinant lacritin, including the step of culturing the lacritin-expressing cell in a medium, and the step of isolating lacritin in the culture supernatant, and an agent for treating ocular disease containing the lacritin-expressing cell.

## Description

### Technical Field

The present invention relates to cells that highly express lacritin. Specifically, the present invention relates to a means of supplying lacritin for use as an active ingredient in the treatment of ocular diseases such as dry eyes.

### Background Art

Lacrimal fluid plays various important roles in the eye. For example, known roles of lacrimal fluid include the protection of corneal and conjunctival epithelial cells by keeping the cornea and conjunctiva moistened, prevention of infections, maintenance of the transparency of the cornea, nutritional supply to the cornea and the like. Diseases known to be caused by lacrimal fluid secretion disorder include ocular diseases such as dry eyes; in dry eyes, countermeasures, for example, replenishment of artificial lacrimal fluid, instillation of a highly viscoelastic substance of high moisture retention quality such as hyarulonic acid, and the like are taken. However, these symptomatic therapies can never serve for radical treatment, although they can improve symptoms. Because lacrimal fluid plays various roles as stated above, and is considered to mitigate ocular diseases, there is demand for the development of a substance that acts on the lacrimal gland to promote lacrimation.

Lacritin is a protein identified as a tear secretion promoting factor or a growth-factor-like protein (see patent document 1 and non-patent document 1). For lacritin, the following 1) to 5) are reported:
1) Lacritin has an activity as a growth factor for a corneal epithelial cell and a lacrimal gland acinar cell.
2) Lacritin shows tear protein secretion promoting effect.
3) Lacritin is expressed in a cell derived from tissues such as the lacrimal gland, parotid gland, minor salivary gland, submandibular gland, thyroid gland and corneal epithelium.
4) Eyedrops containing lacritin are likely to be useful in the treatment of ocular diseases such as dry eye syndrome, Sjogren's syndrome, and corneal epithelial wounds.
5) Compounds that bind to lacritin or lacritin receptors can be screened for using a cell expressing a lacritin receptor with a lacritin-dependent calcium signal as an index.

As stated above, great expectations are emerging for the efficacy of lacritin; however, as the situation stands, cells that spontaneously express lacritin or a lacritin receptor are present only in particular limited tissues, and naturally occurring lacritin has a modified sugar chain, but no means has been established for obtaining lacritin having a modified sugar chain in large amounts using animal cells.
Patent document 1: WO02/065943
Non-patent document 1: Sanghi, S. et al., Journal of Molecular Biology 310, pp.127-139 (2001)

### Summary of the Invention

### Problems to Be Solved by the Invention

It is an object of the present invention to provide a means that allows supplying lacritin stably and in large amounts using animal cells.

### Means of Solving the Problems

The present inventors diligently investigated in view of the above-described problems, succeeded in establishing a cell line that expresses an unprecedentedly high amount of lacritin by integrating nucleotides that encode lacritin into a particular vector, and have completed the present invention. Accordingly, the invention of this application is as follows.

[1] A vector comprising a polynucleotide that encodes lacritin, wherein said nucleotide is operatively linked to a cytomegalovirus immediate early enhancer/promoter and an SV40 late polyA signal.
[2] The vector according to [1], wherein the vector further comprises a chimeric intron consisting of an intron of the β globulin gene and an intron of the immunoglobulin gene heavy-chain variable region.
[3] The vector according to [1] or [2], wherein the vector further comprises as a selection marker the neomycin resistance gene.
[4] A lacritin-expressing cell prepared by introducing the vector according to any one of [1] to [3] into an animal cell.
[5] The lacritin-expressing cell according to [4], wherein the animal cell is a human corneal epithelial cell line.
[6] A method of producing a lacritin-expressing cell, comprising the step of introducing the vector according to any one of [1] to [3] into an animal cell.
[7] The method according to [6], wherein the animal cell is a human corneal epithelial cell line.
[8] A method of producing recombinant lacritin, comprising the step of culturing the lacritin-expressing cell according to [4] or [5] in a medium and the step of isolating lacritin in the culture supernatant.
[9] An agent for treating an ocular disease, comprising the lacritin-expressing cell according to [4] or [5].
[10] The agent according to [9], wherein the ocular disease is keratoconjunctival epithelial disorder or dry eyes.
[11] Use of a lacritin-expressing cell prepared by introducing into an animal cell a vector comprising a cytomegalovirus immediate early enhancer/promoter, a polynucleotide that encodes lacritin and an SV40 late polyA signal, and having said nucleotide operatively linked thereto, for producing an agent for treating an ocular disease.
[12] The use according to [11], wherein the vector further comprises a chimeric intron consisting of an intron of the β globulin gene and an intron of the immunoglobulin gene heavy-chain variable region.
[13] The use according to [11] or [12], wherein the vector further comprises as a selection marker the neomycin resistance gene.
[14] The use according to any one of [11] to [13], wherein the ocular disease is keratoconjunctival epithelial disorder or dry eye.
[15] A method of treating an ocular disease, comprising administering to a subject in need of treatment for the ocular disease an effective amount of a lacritin-expressing cell prepared by introducing a vector comprising a cytomegalovirus immediate early enhancer/promoter, a polynucleotide that encodes lacritin and an SV40 late polyA signal, and having said nucleotide operatively linked thereto, into an animal cell.
[16] The method according to [15], wherein the vector further comprises a chimeric intron consisting of an intron of the β globulin gene and an intron of the immunoglobulin gene heavy-chain variable region.
[17] The method according to [15] or [16], wherein the vector further comprises as a selection marker the neomycin resistance gene.
[18] The method according to [15], wherein the ocular disease is keratoconjunctival epithelial disorder or dry eye. Effect of the Invention

When a vector of the present invention is introduced to an animal cell, a lacritin mRNA having polyA added to an RNA transcribed from a cytomegalovirus immediate early enhancer/promoter by means of an SV40 late polyA signal is produced stably and at high levels, so that the vector is useful as a vector capable of highly expressing lacritin in the animal cell. Because a lacritin-expressing cell of the present invention incorporates the aforementioned vector, the cell can be utilized as a source of recombinant lacritin in the form of a cell sheet, or in the form of recombinant lacritin isolated and purified from a large scale culture. According to a method of the present invention for producing a lacritin-expressing cell, it is possible to easily produce cells capable of highly expressing lacritin using a vector of the present invention. Because an agent for treating ocular disease of the present invention comprises the aforementioned lacritin-expressing cell as an active ingredient, the agent is useful in the prevention or treatment of an ocular disease that results from a lack of lacritin.

### Brief Description of the Drawings

FIG. 1 shows results of a comparison by immunoblotting of the amount of secretory lacritin protein expressed.
FIG. 2 shows results of detection by immunoblotting of secretory lacritin protein. Shown on the left side of the image are the molecular weights (kDa) of migration markers.
FIG. 3 is a graph comparing the number of cells between a cell line that highly expresses lacritin and a cell line incorporating an empty vector.
FIG. 4 shows results of an examination by immunoblotting of the expression of mucin 16 in a cell line that highly expresses lacritin and a cell line incorporating an empty vector.
FIG. 5 is a graph showing an effect of a cell line that highly expresses lacritin on corneal wound healing.
FIG. 6 shows results of a comparison by immunoblotting of the amount of secretory lacritin protein expressed. Shown on the left side of the image are the molecular weights (kDa) of migration markers.

### Mode for Carrying out the Invention

A vector of the present invention is a vector comprising a polynucleotide that encodes lacritin, wherein said nucleotide is operatively linked to a cytomegalovirus immediate early enhancer/promoter and an SV40 late polyA signal.

In the present invention, "lacritin" is exemplified by lacritins derived from mammals such as mice, rats, hamsters, guinea pigs, rabbits, dogs, monkeys, and humans, other animals such as birds such as chicken; for example, when use in humans or human cells is intended, human lacritin (see, for example, GenBank/EBI data bank accession numbers NM_033277 (cDNA) and ay005150 (genomic)) is preferable. The lacritin, as far as it promotes the proliferation of corneal epithelial cells or lacrimal gland acinar cells, may be a variant having one or more (for example, 1 to 100, preferably 1 to 50, more preferably 1 to 20, still more preferably 1 to 10) amino acids substituted, added, deleted or modified. It can be determined whether or not a lacritin variant promotes the proliferation of corneal epithelial cells or lacrimal gland acinar cells, by confirming that the cell count increases significantly compared with the case of non-addition of the lacritin variant when the aforementioned cells are continued to be cultured with the addition of the lacritin variant to the medium at a specified concentration (for example, about 1nM). The lacritin may be a lacritin having an epitope such as a histidine (His) tag, Flag tag, or Myc tag added thereto, so as to make it distinguishable from naturally occurring lacritins, or to facilitate its purification.

"A polynucleotide that encodes lacritin" may be any polynucleotide, such as genomic DNA, mRNA or cDNA, that encodes the above-described lacritin. For ligation to the vector, genomic DNA or cDNA is preferred. The aforementioned polynucleotide may be naturally occurring nucleotides obtained by isolation from a lacritin-expressing cell by a conventional method, or may be synthetic nucleotides obtained by amplification using a primer designed on the basis of a base sequence registered with a data bank such as GenBank.

The promoter used in a vector of the present invention is a cytomegalovirus immediate early enhancer/promoter; the enhancer/promoter is incorporated in a commercially available vector, and can be cut out from a commercially available vector (e.g., pcDNA vector (Invitrogen Japan), pCI-neo vector (Promega K.K.)) by a conventional method using a restriction endonuclease and the like, and linked to a polynucleotide that encodes lacritin.

The polyA signal used in a vector of the present invention is an SV40 late polyA signal. The polyA signal is incorporated in a commercially available vector (e.g., pCI-neo vector (Promega K.K.)), and can be cut out from a commercially available vector by a conventional method using a restriction endonuclease and the like, and linked to a polynucleotide that encodes lacritin.

In a vector of the present invention, the polynucleotide that encodes lacritin is operatively linked to the aforementioned cytomegalovirus immediate early enhancer/promoter and SV40 late polyA signal. Here, "operatively linked" refers to a state wherein the polynucleotide that encodes lacritin is linked so that it will be transcribed from the aforementioned enhancer/promoter, and that a polyA tail will be added to the transcript, in the desired cell. Such ligation in the vector can be achieved by a method known per se; for example, a method can be mentioned wherein a polynucleotide that encodes lacritin is cloned into an appropriate restriction endonuclease site within the multicloning sites of a commercially available pCI-neo vector.

A vector of the present invention preferably further comprises an intron sequence. As the intron sequence, a chimeric intron consisting of an intron of the β globulin gene and an intron of the immunoglobulin gene heavy-chain variable region can be mentioned.

A vector of the present invention can further comprise a selection marker gene for transformant cell selection (genes that confer resistance to drugs such as neomycin, tetracycline, ampicillin, kanamycin, hygromycin, and phosphinothricin, genes that complement auxotrophic mutations, and the like). A preferred selection marker gene is the neomycin resistance gene.

Although the backbone vector used as a vector of the present invention is not particularly limited, a plasmid vector is preferable.

As specific vectors used in the present invention, vectors prepared by integrating a polynucleotide that encodes lacritin into the pCI-neo vector (Promega K.K.), the pCMVTNT vector (Promega K.K.), or the Flexi vector (Promega K.K.) and the like can be mentioned; a vector prepared by integrating a polynucleotide that encodes lacritin into the pCI-neo vector is preferable.

A lacritin-expressing cell of the present invention is obtained by introducing a vector of the present invention into a cell.

Examples of cells for vector introduction include cells derived from mammals such as mice, rats, hamsters, guinea pigs, rabbits, dogs, monkeys, and humans, and other animals such as birds such as chicken. The cells may be cells such as lacrimal gland cells, parotid gland cells, minor salivary gland cells, submandibular gland cells, thyroid gland cells, corneal cells, thymic cells, and conjunctival cells. The cells may also be primary culture cells, a cell line derived from primary culture cells, cells obtained by culturing undifferentiated cells such as stem cells (for example, differentiated cells), a commercially available cell line, a cell line available from a cell bank, and the like. Considering human application and graft utilization, the cells for vector introduction are preferably human corneal epithelial cell lines. Human corneal epithelial cell lines can be established from human corneal cells according to a method described in Invest Ophthalmol Vis Sci. 1995, 36, 614-621.

Useful methods of introducing a vector of the present invention into a cell include methods known per se, for example, electroporation, calcium phosphate precipitation, microinjection, methods using lipids such as liposomes or cationic lipids, and the like. The vector may or may not be incorporated in the genome of the cell for vector introduction. Given that a selection marker is contained in a vector of the present invention, it is possible to easily select cells incorporating the vector of the present invention, by setting culture conditions suitable for the selection marker.

After introduction of a vector of the present invention to cells, a lacritin-expressing cell is cloned by a conventional method, whereby a lacritin-expressing cell of the present invention is obtained. The present invention provides this method of producing a lacritin-expressing cell.

An agent for treating ocular disease of the present invention comprises a lacritin-expressing cell of the present invention. When a lacritin-expressing cell is contained, the agent for treating ocular disease can comprise an optionally chosen medium and the like. As the optionally chosen medium, for example, cell culture medium, medium additives and the like can be mentioned.

An agent for treating ocular disease of the present invention, which comprises a lacritin-expressing cell, can also be transplanted to an animal such as a mammal. In this case, a lacritin-expressing cell may be provided as a cell sheet, a cell layer or a tissue equivalent for transplantation (hereinafter, abbreviated as "graft" as required). Technologies concerning graft preparation are described in detail in, for example, WO03/084431, WO03/026712, WO03/009783, WO00/2955 WO96/13974, JP-A-2003-38170, JP-A-2001-161353, US20020039788 and the like. Also, a lacritin-expressing cell enables xenogeneic transplantation, allogeneic transplantation or syngeneic transplantation depending on the kind of transplantation and the kind of animal intended to receive the graft. The lacritin-expressing cell of the present invention can be used as an agent for preventing/treating an ocular disease for which administration of lacritin is desired. Ocular diseases to which the above-described cell is desirably applied include dry eye syndrome, Sjogren's syndrome, keratoconjunctival epithelial disorder, meibomian gland dysfunction, keratoconjunctivitis sicca, blepharitis, Stevens-Johnson syndrome, and dry eyes associated with VDT (visual display terminal) work. Preferably, the ocular disease is keratoconjunctival epithelial disorder or dry eye.

A lacritin-expressing cell of the present invention can be utilized as a corneal wound healing promoter.

In another embodiment, the lacritin-expressing cell of the present invention can be a promoter of cell proliferation. As examples of cells whose proliferation is promoted by the cell, ocular cells such as corneal epithelial cells, corneal endothelial cells, and lacrimal gland cells; acinar cells such as lacrimal gland acinar cells, salivary gland acinar cells, and thymic acinar cells, and the like can be mentioned; corneal epithelial cells are preferable.

In another embodiment, the lacritin-expressing cell of the present invention can be a promoter of the secretion of secretes such as lacrimal fluid. For example, the cell makes it possible to increase the potential for lacrimation from lacrimal gland cells by highly expressing lacritin.

In another embodiment, the lacritin-expressing cell of the present invention can be a promoter of mucin secretion. The cell makes it possible to protect the corneal epithelium by promoting mucin secretion and allowing the secreted mucin to play a role in retaining a lacrimal fluid layer on the eye surface.

The present invention provides a method of producing recombinant lacritin, comprising using the aforementioned lacritin-expressing cell. The method of production comprises the step of culturing the aforementioned lacritin-expressing cell in a medium, and the step of isolating lacritin in the culture supernatant.

### Step of culturing a lacritin-expressing cell in a medium

The cell culture can be performed using a medium, nutritional components, culturing environmental factors such as temperature, humidity, and CO₂ concentration, and culturing time, which are suitable for the lacritin-expressing cell and chosen as appropriate. When the lacritin-expressing cell is a human corneal epithelial cell line, the cell culture can also be performed using a medium, nutritional components, culturing environmental factors such as temperature, humidity, and CO₂ concentration, and culturing time, which are suitable for the cell and chosen as appropriate. For example, the cell can be cultured under conditions of 37°C, 5% CO₂, 100% humidity and the like.

### Step for isolating lacritin in culture supernatant

In the aforementioned culturing step, lacritin is secreted from the lacritin-expressing cell into the medium. In this step, the medium is recovered, and lacritin is isolated from the culture supernatant. Usually, the culture supernatant is obtained via centrifugation. Lacritin can be purified as appropriate from the culture supernatant by a method known per se, for example, methods based on differences in solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography and use of lacritin antibody; methods based on differences in hydrophobicity, such as reverse phase high performance liquid chromatography; methods based on differences in isoelectric point, such as isoelectric focusing; combinations thereof, and the like.

The recombinant lacritin thus obtained can be prepared along with a pharmaceutically acceptable carrier by a conventional method, and can be provided as an agent for treating an ocular disease in need of lacritin.

Examples of the pharmaceutically acceptable carrier include, but are not limited to, excipients such as sucrose, starch, mannitol, sorbitol, lactose, glucose, cellulose, talc, calcium phosphate and calcium carbonate; binders such as cellulose, methylcellulose, hydroxypropylcellulose, polypropylpyrrolidone, gelatin, acacia, polyethylene glycol, sucrose and starch; disintegrants such as starch, carboxymethylcellulose, hydroxypropyl starch, sodium-glycol-starch, sodium hydrogen carbonate, calcium phosphate and calcium citrate; lubricants such as magnesium stearate, Aerosil, talc and sodium lauryl sulfate; preservatives such as sodium benzoate, sodium hydrogen sulfite, methyl paraben and propyl paraben; stabilizers such as citric acid, sodium citrate and acetic acid; suspending agents such as methylcellulose, polyvinylpyrrolidone and aluminum stearate; dispersing agents such as surfactants; diluents such as water and physiological saline; base waxes such as cacao butter, polyethylene glycol and kerosene; and the like.

Although the dosage of the recombinant lacritin varies depending on the seriousness of illness, recipient animal species, the recipient's drug tolerance, body weight, age, and the like, and cannot be generalized, it is normally about 0.001 to about 500 mg/kg, as the amount of active ingredient per day for an adult.

### Examples

The present invention is hereinafter described in further detail with reference to the following Examples, to which, however, the present invention is not limited.

### Example 1 Construction of pCI-neo vector comprising the human lacritin gene

The sequence from the start codon to the stop codon of the human lacritin gene was amplified using the PCR technique. PCR was performed by repeating heat treatment at 94°C for 30 seconds, at 55°C for 30 seconds, and at 72°C for 1 minute, in 35 cycles, whereby a lacritin gene fragment was amplified from a cDNA. Previously, a sequence comprising an XhoI restriction site was inserted into the primer on the Forward side (5'- GGT GGT TCT CGA GGC CAC CAT GAA ATT TAC CAC TCT CCT CT-3': SEQ ID NO:1), and a sequence comprising an XbaI restriction site was inserted into the primer on the Reverse side (5'-GGT GGT T TCT AGA AT CAG CTC ATG CCC ATG GTT TTA ATA GAC-3': SEQ ID NO:2); the human lacritin gene fragment amplified was cleaved with each restriction endonuclease, after which the fragment was integrated into the pCI-neo mammalian vector (Promega K.K.). Thereafter, to obtain a large amount of a vector incorporating lacritin, Escherichia coli TOP10 was transformed with the aforementioned vector comprising the lacritin gene, using One Shot competent cells (Invitrogen Japan K.K.). Vector purification from Escherichia coli was performed using Plasmid purification kits (QIAGEN K.K.).
The pCI-neo vector comprises a cytomegalovirus immediate early enhancer/promoter as the promoter, comprises an SV40 late polyA signal as the polyA signal, and further comprises a chimeric intron consisting of an intron of the β globulin gene and an intron of the immunoglobulin gene heavy-chain variable region and the neomycin resistance gene.

### Comparative Example 1 Construction of pcDNA3.1 TOPO vector comprising the human lacritin gene

The human lacritin gene fragment amplified in Example 1 was integrated into the pcDNA3.1 TOPO vector (Invitrogen Japan K.K.) by means of the TA cloning method. Thereafter, in the same manner as Example 1, Escherichia coli TOP10 was transformed with the vector.
The pcDNA3.1 TOPO vector comprises a cytomegalovirus immediate early enhancer/promoter as the promoter, comprises a BGH polyadenylation signal as the polyA signal, and further comprises the neomycin resistance gene.

### Example 2 Comparison of amounts of lacritin protein secreted between different vectors

Using the FreeStyle 293-cell expression kit (Invitrogen Japan K.K.), amounts of lacritin protein secreted were compared between the vectors prepared in Example 1 and Comparative Example 1. First, each vector comprising the lacritin gene was introduced to 293-cells, and the cells were subjected to shaking culture for 48 hours (FreeStyle 293-cell expression kit). After completion of the cultivation, the medium was recovered and concentrated using an ultrafiltration filter. The concentrated culture broth was electrophoresed with 12% NuPAGE gel (Invitrogen Japan K.K.) (MES buffer solution, 200V, 35 minutes); thereafter using a blotting buffer (20% MeOH, Tris-glycine buffer), blotting was performed (100V, 90 minutes). The marker used was the Precision Plus Blue Standard (Bio-Rad Laboratories K.K.). A TTBS containing 5% skimmed milk was used for membrane blocking and antibody dilution. The primary antibody used was an anti-lacritin antibody (manufactured by Takara Bio Inc.) in 2000-fold dilution; the secondary antibody used was an anti-rabbit IgG-HRP conjugate (Santa Cruz K.K.) in 5000-fold dilution. Detection was achieved using ECL plus (GE healthcare bioscience K.K.); images were captured using Chemi-Doc (Bio-Rad Laboratories K.K.). The images captured were checked using Scion Image to determine the intensities thereof.

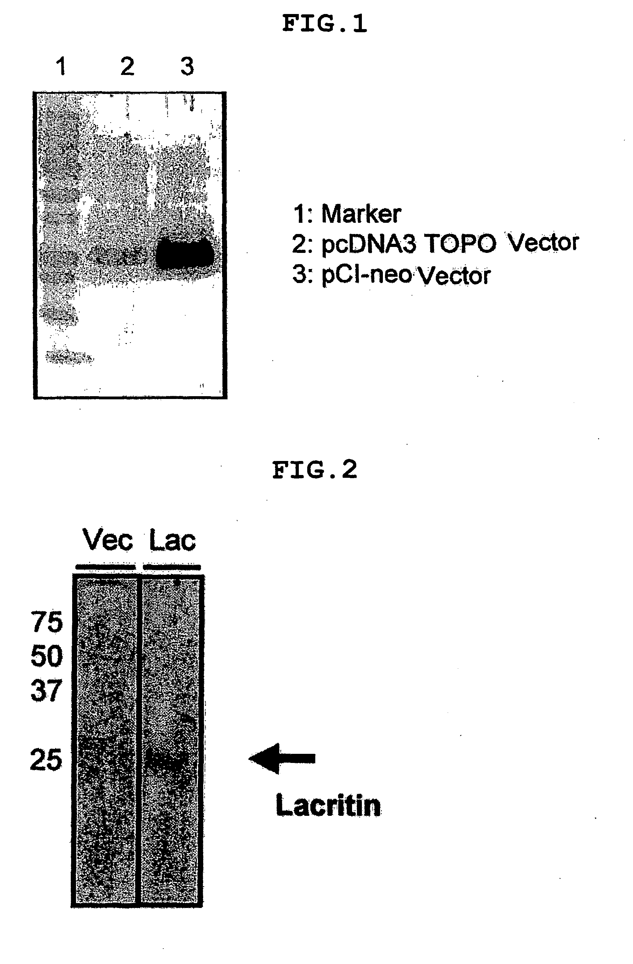

As a result, as shown in FIG. 1, in the cells incorporating lacritin introduced thereto using the pCI-neo vector prepared in Example 1, compared with the cells incorporating lacritin introduced thereto using the pcDNA3.1 TOPO vector prepared in Comparative Example 1, the amount of lacritin protein secreted increased. The amount of lacritin protein secreted from the cells incorporating the pCI-neo vector was about 13 times as much as the amount secreted from the cells incorporating the pcDNA3.1 TOPO vector.

### Example 3 Establishment of cell line that highly expresses lacritin

Using the vector prepared in Example 1, a cell line that stably highly expresses lacritin was established. The vector prepared in Example 1 was introduced to a human corneal epithelial cell line (HCE-T: can be prepared by a method described in Invest Ophthalmol Vis Sci. 1995, 36, 614-621), using Lipofectamine (Invitrogen Japan K.K.). Thereafter, by culturing the cell in the presence of Geneticin (Invitrogen Japan K.K.), a cell line having lacritin introduced into the chromosome thereof was obtained.

### Example 4 Expression of lacritin protein secreted from highly expressing cell line

To confirm the secretion of lacritin protein from the cell line that highly expresses lacritin established in Example 3, confirmation was performed using immunoblotting.
1×10⁷ cells of the lacritin-expressing cell line established in Example 3 were seeded to a 225cm² flask; the following day, the medium was replaced with Opti-MEM (Invitrogen Japan K.K.), and the cells were cultured for 48 hours. After completion of the cultivation, the medium was recovered, and the medium was concentrated 100 fold using an ultrafiltration filter. The concentrated culture broth was electrophoresed with 12% NuPAGE gel (MES buffer solution, 200V, 35 minutes); thereafter, using a blotting buffer (20% MeOH, Tris-glycine buffer), blotting was performed (100V, 90 minutes). A TTBS containing 5% skimmed milk was used for membrane blocking and antibody dilution. The primary antibody used was an anti-lacritin antibody (Takara Bio Inc.) in 2000-fold dilution for an antibody reaction; the secondary antibody used was an anti-rabbit IgG-HRP conjugate (Santa Cruz K.K.) in 5000-fold dilution. Detection was achieved using ECL plus (GE healthcare bioscience K.K.); images were captured using Chemi-Doc (Bio-Rad Laboratories K.K.).

As a result, as shown in FIG. 2, the high-expression cell line having the lacritin gene introduced thereto using the pCI-neo vector produced a band indicating the secretion of lacritin protein at a position for 25 kDa. Meanwhile, in the cells incorporating the pCI-neo vector only, no band was detected in the culture broth.

### Example 5 Proliferation experiments for cells that highly express lacritin

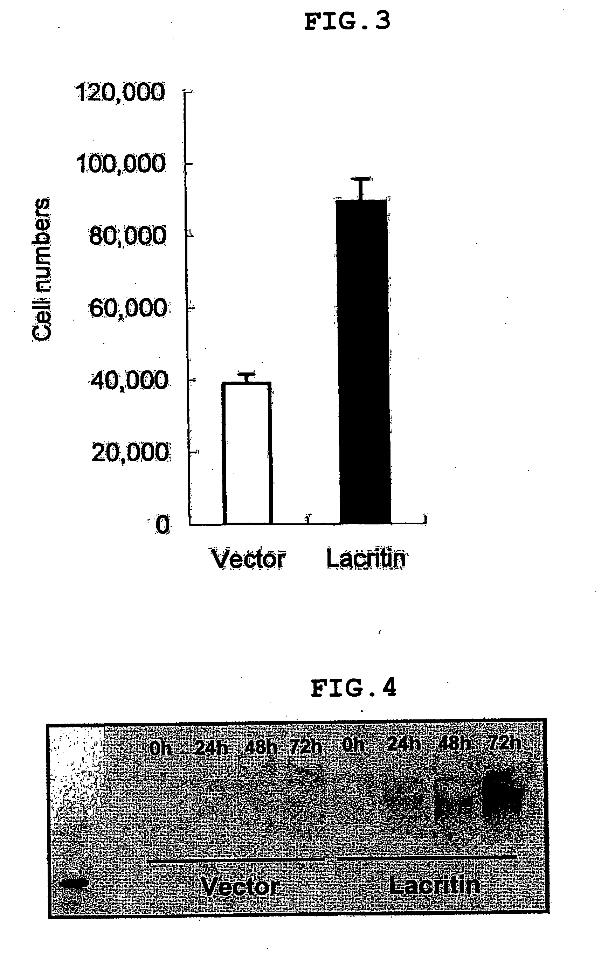

To confirm the function of lacritin secreted from cells that highly express lacritin, a cell proliferation effect was examined. The lacritin-expressing cell line established in Example 3 (5×10³ cells) was seeded to a 24-well plate. The following day, the medium was replaced with a Geneticin-containing EpiLife medium (Kurabo Industries, Ltd.), and the study was commenced. Cultivation was performed for 6 days in total; on day 3 in the midst of the cultivation, the culture broth was once replaced with a fresh supply. Cell counts were taken using a blood cell counting chamber on day 6 of cultivation. Results are shown in FIG. 3.

As a result, the cell line that highly expresses lacritin was found to possess higher proliferating capability than that of the cell line incorporating the vector only. Consequently, it can be thought that the proliferating capability of corneal epithelial cells was increased by the secreted protein that acted on the cells.

### Example 6 Test on enhancement of mucin 16 expression

Mucin is an important protein for the maintenance of the quantitative and qualitative consistency of lacrimal fluid. Hence, using a cell line that highly expresses lacritin, the effect of lacritin protein on the expression of mucin 16, a membrane mucin, was examined.
The lacritin-expressing cell line established in Example 3 (2×10⁵ cells) was seeded to a 6-well plate; on the day before the cells became confluent, the culture broth was replaced with serum-free DMEM/F12 (Invitrogen Japan K.K.). The following day, the culture broth was again replaced with a fresh medium, after which the cells were cultured for 3 days. Thereafter, the cells were lysed using an RIPA buffer solution (50mM Tris, 0.1% SDS, 0.5% deoxycholate, 1% NP-40, 150mM NaCl, complete protein inhibitor cocktail (Rhoche)), and disrupted using a sonicator; this was followed by centrifugation at 12000 rpm for 15 minutes at 4°C, and soluble protein was recovered. After the protein concentration of the sample recovered was measured, 40-50 µg of the soluble protein was electrophoresed with 4-12% NuPAGE gel (MOPS buffer, 200V, 70 minutes); thereafter, using a blotting buffer (0.02% SDS, 20% MeOH, Tris-glycine buffer), blotting was performed at 100V for 60 minutes. A TTBS containing 5% skimmed milk was used for membrane blocking and antibody dilution. The primary antibody used was OC125 (Dako Japan Co., Ltd.) in 1000-fold dilution for an antibody reaction; the secondary antibody used was an anti-mouse IgG-HRP conjugate (Santa Cruz K.K.) in 5000-fold dilution. Detection was achieved using ECL plus (GE healthcare bioscience K.K.); images were captured using Chemi-Doc.

As a result, as shown in FIG. 4, the amount of mucin 16 expressed was higher at all time points in the cell line that highly expresses lacritin, compared with the cell line incorporating the empty vector. Hence, it can be thought that the expression of mucin 16 was enhanced because the secreted lacritin acted on corneal epithelial cells.

### Example 7 Corneal wound healing experiments

Using a corneal wound healing model, the wound healing effect of a cell line that highly expresses lacritin was examined. For plate pre-treatment, a plate seal (SANPLATEC Corporation), cut into a 1-inch circle, was applied to a 24-well plate in the center of the base thereof, after which the plate was irradiated with UV for 2 to 3 hours for sterilization. Thereafter, using DMEM/F12/5% FBS medium, the lacritin-expressing cell line established in Example 3 was suspended to obtain a cell density of 5 to 16×10⁴ cells/mL, and the cells were seeded to the pre-treated 24-well plate at 500 µL per well (2.5 to 8×10⁴ cells/well). Thereafter, the cells were cultured at 37°C, 5% CO₂ for 3 days until they reached confluency. After the cells became confluent, the plate seal that was previously applied to the 24-well plate was carefully removed. Next, the culture supernatant was removed, after which serum-free, Geneticin-containing DMEM/F12 was dispensed at 500 µL per well. Thereafter, the cells were cultured for 3 days under conditions of 37°C, 5% CO₂. To evaluate the wound healing effect of the cells, the cells were fixed with 10% formalin, and thereafter stained with 1% Crystal Violet; the area of the residual epithelial defect was measured on computerized images.

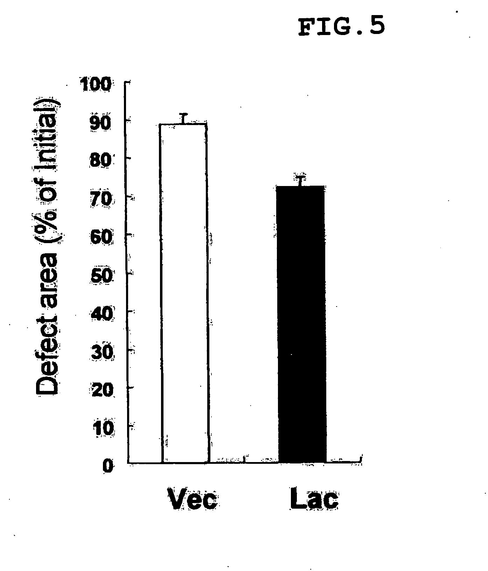

As a result, as shown in FIG. 5, in the cell line incorporating the empty vector, the defective area accounted for about 90%; in the cell line that highly expresses lacritin, the defective area accounted for about 70%. Hence, it can be thought that the secreted lacritin acted on corneal epithelial cells to exhibit the corneal wound healing promoting effect.

### Comparative Example 2 Construction of pBApo-CMV vector comprising the human lacritin gene

The sequence from the start codon to the stop codon of the human lacritin gene was amplified by the PCR technique. PCR was performed by repeating heat treatment at 94°C for 30 seconds, at 55°C for 30 seconds, and at 68°C for 1 minute, in 25 cycles, whereby a lacritin gene fragment was amplified from a cDNA. Previously, a sequence comprising a BamHI restriction site was inserted into the primer on the Forward side (5'- AAC CGG ATC CGC CAC CAT GAA ATT TAC CAC TCT CCT CT-3': SEQ ID NO:3), and a sequence comprising a Hind III restriction site was inserted into the primer on the Reverse side (5'- CCC CAA GCT TCA TGC CCA TGG TTT TAA TAG AC-3': SEQ ID NO:4); the human lacritin gene fragment amplified was cleaved with each restriction endonuclease, after which the fragment was integrated into the pBApo-CMV Neo vector (Takara Bio Inc.). Thereafter, to obtain a large amount of a vector incorporating lacritin, Escherichia coli DH5α was transformed with the aforementioned vector comprising the lacritin gene, using MAX Efficiency DH5α competent cells (Invitrogen Japan K.K.). Vector purification from the Escherichia coli was performed using the Invitrogen PureLink HiPure Plasmid Gigaprep Kit (Invitrogen Japan K.K.).
The pBApo-CMV vector comprises a cytomegalovirus immediate early enhancer/promoter as the promoter, comprises a polyA signal of herpes simplex virus thymidine kinase as the polyA signal, and further comprises the neomycin resistance gene.

### Comparative Example 3 Construction of pEF6 TOPO vector comprising the human lacritin gene

The human lacritin gene fragment amplified in Comparative Example 2 was integrated into the pEF6 TOPO vector (Invitrogen Japan K.K.) by utilizing the TA cloning method. Thereafter, to obtain a large amount of a vector incorporating lacritin, Escherichia coli TOP10 was transformed with the pEF6 TOPO vector comprising the lacritin gene, using One shot TOP10 competent cells (Invitrogen Japan K.K.).
The pEF6 TOPO vector comprises an hEF-1α gene promoter as the promoter, comprises a BGH polyadenylation signal as the polyA signal, and further comprises the blasticidin resistance gene.

### Example 8 Comparison of amounts of lacritin protein secreted among different vectors

Using the FreeStyle 293-cell expression kit (Invitrogen Japan K.K.), amounts of lacritin protein expressed were compared among the vectors prepared in Example 1, Comparative Example 2 and Comparative Example 3. First, each vector comprising the lacritin gene was introduced to 293-cells, and the cells were subjected to shaking culture for 48 hours and 72 hours (FreeStyle 293-cell expression kit). After completion of the cultivation, the medium was recovered and concentrated using an ultrafiltration filter. The concentrated culture broth was electrophoresed with 12% NuPAGE gel (Invitrogen Japan K.K.) (MES buffer solution, 200V, 35 minutes); thereafter, using a blotting buffer (20% MeOH, Tris-glycine buffer), blotting was performed (100V, 90 minutes). A TTBS containing 5% skimmed milk was used for membrane blocking and antibody dilution. The primary antibody used was an anti-lacritin antibody (manufactured by Takara Bio Inc.) in 2000-fold dilution for an antibody reaction; the secondary antibody used was an anti-rabbit IgG-HRP conjugate (Santa Cruz K.K.) in 5000-fold dilution. Detection was achieved using ECL plus (GE healthcare bioscience K.K.); images were captured using Chemi-Doc (Bio-Rad Laboratories K.K.).

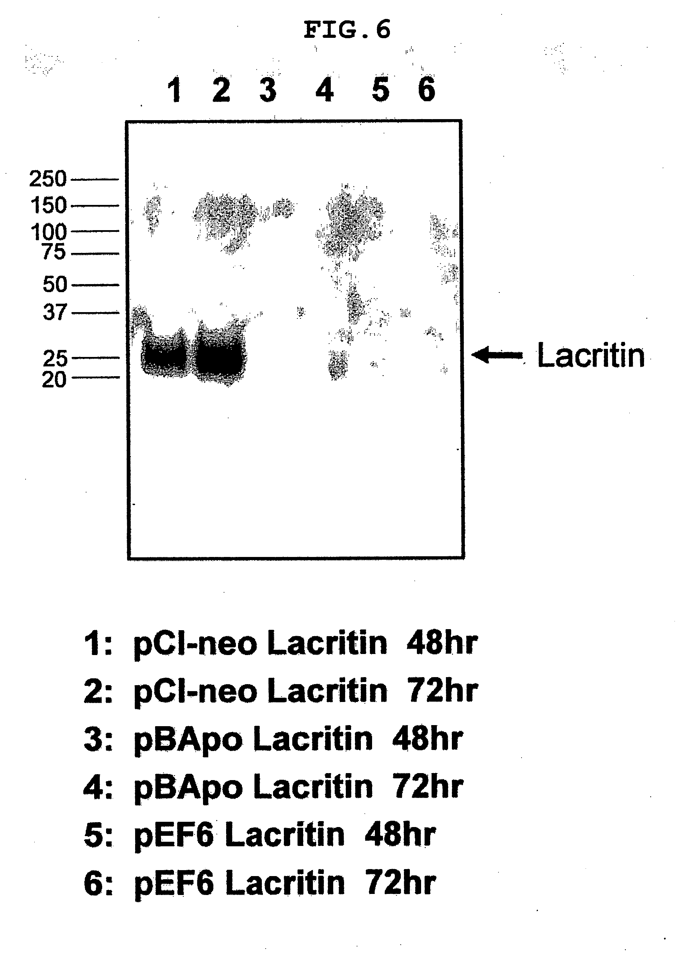

As a result, as shown in FIG. 6, a band for lacritin was detected in the cells incorporating lacritin introduced using the pCI-neo vector prepared in Example 1; whereas in the cells incorporating lacritin introduced using the pBApo-CMV vector and pEF6 TOPO vector prepared in Comparative Examples 2 and 3, no band of lacritin was detected at all. Hence, it was found that when lacritin protein was expressed using the pCI-neo vector, the amount secreted increased remarkably.

### Industrial Applicability

A vector of the present invention is useful as a vector capable of highly expressing lacritin in animal cells. A lacritin-expressing cell of the present invention can be utilized as a source of recombinant lacritin in the form of a cell sheet, or in the form of recombinant lacritin isolated and purified from a large scale culture. A method of producing a lacritin-expressing cell of the present invention makes it possible to easily produce cells capable of highly expressing lacritin using a vector of the present invention. An agent for treating ocular disease of the present invention is useful in the prevention or treatment of an ocular disease due to a lack of lacritin because it comprises the aforementioned lacritin-expressing cell as an active ingredient.

This application is based on a patent application No. 2007-049936 filed in Japan (filing date: February 28, 2007), the contents of which are incorporated in full herein by this reference.

## Claims

1. A vector comprising a polynucleotide that encodes lacritin, wherein said nucleotide is operatively linked to a cytomegalovirus immediate early enhancer/promoter and an SV40 late polyA signal.

2. The vector according to claim 1, wherein the vector further comprises a chimeric intron consisting of an intron of the β globulin gene and an intron of the immunoglobulin gene heavy-chain variable region.

3. The vector according to claim 1 or 2, wherein the vector further comprises as a selection marker the neomycin resistance gene.

4. A lacritin-expressing cell prepared by introducing the vector according to any one of claims 1 to 3 into an animal cell.

5. The lacritin-expressing cell according to claim 4, wherein the animal cell is a human corneal epithelial cell line.

6. A method of producing a lacritin-expressing cell, comprising the step of introducing the vector according to any one of claims 1 to 3 into an animal cell.

7. The method according to claim 6, wherein the animal cell is a human corneal epithelial cell line.

8. A method of producing recombinant lacritin, comprising the step of culturing the lacritin-expressing cell according to claim 4 or 5 in a medium and the step of isolating lacritin in the culture supernatant.

9. An agent for treating an ocular disease, comprising the lacritin-expressing cell according to claim 4 or 5.

10. The agent according to claim 9, wherein the ocular disease is keratoconjunctival epithelial disorder or dry eyes.

11. Use of a lacritin-expressing cell prepared by introducing into an animal cell a vector comprising a cytomegalovirus immediate early enhancer/promoter, a polynucleotide that encodes lacritin and an SV40 late polyA signal, and having said nucleotide operatively linked thereto, for producing an agent for treating an ocular disease.

12. The use according to claim 11, wherein the vector further comprises a chimeric intron consisting of an intron of the β globulin gene and an intron of the immunoglobulin gene heavy-chain variable region.

13. The use according to claim 11 or 12, wherein the vector further comprises as a selection marker the neomycin resistance gene.

14. The use according to any one of claims 11 to 13, wherein the ocular disease is keratoconjunctival epithelial disorder or dry eye.

15. A method of treating an ocular disease, comprising administering to a subject in need of treatment for the ocular disease an effective amount of a lacritin-expressing cell prepared by introducing a vector comprising a cytomegalovirus immediate early enhancer/promoter, a polynucleotide that encodes lacritin and an SV40 late polyA signal, and having said nucleotide operatively linked thereto, into an animal cell.

16. The method according to claim 15, wherein the vector further comprises a chimeric intron consisting of an intron of the β globulin gene and an intron of the immunoglobulin gene heavy-chain variable region.

17. The method according to claim 15 or 16, wherein the vector further comprises as a selection marker the neomycin resistance gene.

18. The method according to claim 15, wherein the ocular disease is keratoconjunctival epithelial disorder or dry eye.


SEQUENCE LISTING

<110> Senju Pharmaceutical Co., Ltd.

<120> CELL CAPABLE OF EXPRESSING LACRITIN AT HIGH LEVEL

<130> 091182

<150> JP 2007-049936
<151> 2007-02-28

<160> 4

<170> PatentIn version 3.1

<210> 1
<211> 41
<212> DNA
<213> Artificial

<220>
<223> primer

<400> 1
ggtggttctc gaggccacca tgaaatttac cactctcctc t 41

<210> 2
<211> 42
<212> DNA
<213> Artificial

<220>
<223> primer

<400> 2
ggtggtttct agaatcagct catgcccatg gttttaatag ac 42

<210> 3
<211> 38
<212> DNA
<213> Artificial

<220>
<223> primer

<400> 3
aaccggatcc gccaccatga aatttaccac tctcctct 38

<210> 4
<211> 32
<212> DNA
<213> Artificial

<220>
<223> primer

<400> 4
ccccaagctt catgcccatg gttttaatag ac 32

<110>  Senju Pharmaceutical Co., Ltd.

<120>  A cell highly expressing lacritin

<130>  091182

<150>  JP 2007-049936
<151>  2007-02-28

<160>  4

<170>  PatentIn version 3.1

<210>  1
<211>  41
<212>  DNA
<213>  Artificial

<220>
<223>  primer

<400>  1
ggtggttctc gaggccacca tgaaatttac cactctcctc t                          41


<210>  2
<211>  42
<212>  DNA
<213>  Artificial

<220>
<223>  primer

<400>  2
ggtggtttct agaatcagct catgcccatg gttttaatag ac                         42


<210>  3
<211>  38
<212>  DNA
<213>  Artificial

<220>
<223>  primer

<400>  3
aaccggatcc gccaccatga aatttaccac tctcctct                              38


<210>  4
<211>  32
<212>  DNA
<213>  Artificial

<220>
<223>  primer

<400>  4
ccccaagctt catgcccatg gttttaatag ac                                    32